(19) Europäisches Patentamt / European Patent Office / Office européen des brevets

(11) **EP 4 378 436 B1**

(12) **EUROPEAN PATENT SPECIFICATION**

(45) Date of publication and mention
of the grant of the patent:
**27.08.2025  Bulletin 2025/35**

(21) Application number: **22210316.0**

(22) Date of filing: **29.11.2022**

(51) International Patent Classification (IPC):
***A61F 13/15*** *(2006.01)*

(52) Cooperative Patent Classification (CPC):
**A61F 13/15658;** A61F 2013/530489

(54) **APPARATUS FOR DISTRIBUTING SUPERABSORBENT PARTICLES ONTO A SUBSTRATE**

VORRICHTUNG ZUR VERTEILUNG VON SUPERABSORBIERENDEN TEILCHEN AUF EIN SUBSTRAT

APPAREIL POUR DISTRIBUER DES PARTICULES SUPERABSORBANTES SUR UN SUBSTRAT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**05.06.2024  Bulletin 2024/23**

(73) Proprietors:
• **Ontex BV
9255 Buggenhout (BE)**
• **Ontex Group NV
9320 Erembodegem (BE)**

(72) Inventor: **Heege, Thomas
56761 Düngenheim (DE)**

(74) Representative: **Saurat, Thibault
Ontex BV
Legal Department
Korte Keppestraat 21
9320 Erembodegem (BE)**

(56) References cited:
**JP-A- 2009 112 347     US-A1- 2005 234 412
US-A1- 2014 027 943**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## TECHNICAL FIELD

The invention pertains to the technical field of absorbent articles and the method and apparatus to form the absorbent core of said absorbent article such as disposable diapers and the like. In particular, the invention concerns an apparatus suitable for distributing superabsorbent particles homogeneously onto a substrate and as well as an apparatus suitable for forming moulded absorbent material deposit structures to be used as absorbent cores for absorbent articles comprising said apparatus suitable for distributing superabsorbent particles homogeneously onto a substrate. The present disclosure also relates to a method for forming moulded absorbent material deposit structures to be used as absorbent cores using said apparatus.

## BACKGROUND

As known, disposable absorbent articles such as diapers or training pants for babies, sanitary napkins or towels or products for adult incontinence, comprises an absorbent core arranged in between a liquid permeable topsheet and a liquid impermeable backsheet with eventually an acquisition distribution layer (ADL) in between the topsheet and the absorbent core. The general purpose of such absorbent articles is to absorb, distribute and store various types of body exudates, while providing a high level of comfort and sense of dryness to the wearer during use of the absorbent article. In order to do so, the disposables typically comprise an absorbent core, or absorbent body, which is capable of quickly absorbing and retaining the exudates. The absorbent core comprises absorbent material such as cellulosic fibres or fluff or fluff pulp and/or of particles of superabsorbent material (SAP). The absorbent material is usually contained, encapsulated or enclosed in a core wrap. The core wrap can comprise one core wrap sheet extending above and under and on the side of the absorbent material thereby fully or at least partially covering the absorbent material. Alternatively, the core wrap can comprise two core wrap sheet, an upper core wrap and a lower absorbent core wrap, the absorbent material between arranged in between. In recent years, more and more absorbent cores comprise at least one channel substantially free of absorbent material. The at least one channel is formed by adhering the lower core wrap sheet to the upper core wrap sheet, or the portion of the sheet above to the portion of the sheet under when the core wrap comprises one single sheet.

The process and apparatus commonly used for the formation of absorbent bodies is generally described below.

The absorbent cores are manufactured using an apparatus comprising a defibrating device, such as a mill or more specifically a hammermill, which defibers, or pulverizes, a sheet of pulp material, resulting in pulp fibers. Additionally, the apparatus comprises a tube feeding superabsorbent particles within said duct and superabsorbent particles are discharged into said duct. In this way, the superabsorbent particles and the pulp fibers flow together within the duct. An airflow conveys the pulp fibers and superabsorbent particles to a forming pocket where the absorbent fibers and superabsorbent particles are deposited thereby forming a moulded absorbent material deposit structure.

It is required that the absorbent material, in particular the superabsorbent particles, have an adequate and uniform distribution, since the functionality of the absorbent core in the disposable article will depend on that.

Documents US2005/0234412, US2011/0166541 and US2014/0027943 all disclose devices for distributing superabsorbent polymer where a valve, nozzle or barrier is arranged at the end of a tube, said nozzle or valve deflecting all the superabsorbent polymer to have an allegedly homogenous distribution.

The above publications have made attempts in providing substantially uniform distribution of the particulate super absorbent material. However, none of the foregoing appears to have adequately addressed the problem and there remains a need to have a more uniform distribution of superabsorbent particles. It is thus an object of the present invention to provide a method and apparatus that solves these problems by making the distribution of the super absorbent material within the forming pockets more uniform. An apparatus for distributing superabsorbent particles is also known from JP 2009 112347 A.

## SUMMARY

The present invention pertains to an apparatus for distributing superabsorbent particles onto a substrate, the apparatus comprising a discharge pipe for conveying a stream comprising superabsorbent particles and a first airflow, said discharge pipe comprising a conveying section and an outlet nozzle, said outlet nozzle comprising a straight portion preferably arranged in the continuity of the conveying section and a deflecting portion characterized in that the deflecting portion comprises deflecting means arranged in a way that only a portion of the stream of superabsorbent particle and first airflow is deflected and the other portion of said stream is not deflected, creating a crossflow between the portion of the said stream that is deflected and the portion of said stream that is not deflected, preferably at the outlet of the discharging pipe.

According to an embodiment, the deflecting means define a first angle between the conveying section and the deflection portion, said first angle being comprised between 91° and 179°, preferably between 120° and 175°, more preferably between 150° and 170°.

According to an embodiment, the discharge pipe extends longitudinally, transversally and vertically, said outlet nozzle comprises a top wall, a bottom wall, a first and second side wall, each wall comprising a proximal end connected to the conveying section and a distal end arranged at the outlet of the discharging pipe, the distal end of the top wall extending longitudinally beyond the distal end of the bottom wall and/or the distal end of the first side wall extending longitudinally beyond the distal end of the second side wall.

According to an embodiment, the discharge pipe further comprises a first feeding tube conveying a first airflow, a second feeding tube conveying superabsorbent particles and a venturi section arranged at the junction between the first and second feeding tubes and the conveying section, the first and second feeding tubes and the conveying section all being fluidically connected, first and second feeding tubes being arranged upstream of the conveying section with respect to the direction of flow.

According to an embodiment, the venturi section is formed by the second feeding tube partially extending within first feeding tube and reducing the cross-sectional area of the discharge pipe for the first airflow, meaning for the passage of the first airflow.

According to an embodiment, the first feeding tube and the second feeding tube define a third angle, said third angle being comprised between 0° and 90°, preferably between 15° and 60°.

According to an embodiment, the outlet nozzle comprises a rectangular transversal cross section and the cross-sectional area of outlet nozzle is lesser than the cross-sectional area of the conveying section.

The present disclosure also relates to an apparatus suitable for forming moulded absorbent material deposit structures to be used as absorbent cores for absorbent articles, the apparatus comprising:

- a forming unit comprising: a forming drum comprising an outer circumferential surface, a plurality of forming pockets arranged on the outer circumferential surface of said forming drum, each forming pocket comprising a mould cavity; and
- a feeding unit comprising:

  ○ a defibrating station configured to generate absorbent fibers and a second airflow,
  ○ a discharging conduit defined by a housing fluidically connecting the defibrating station and the forming unit through which the plurality of absorbent fibers and a second airflow flow,
  ○ an apparatus for distributing superabsorbent particles as described hereabove.

According to the present disclosure, the discharge pipe extends partially within the discharging conduit, the outlet nozzle being arranged within the discharging conduit in a way that the discharge pipe sprays superabsorbent particles into the discharging conduit and into the mould cavity of the forming pockets.

According to an embodiment, the feeding unit comprises two discharging conduits, each conduit being fluidically connected to the defibrating station and each conduit comprising a discharge pipe extending partially within its respective discharging conduit, the outlet nozzle of each pipe being arranged within its respective discharging conduit.

According to an embodiment, the apparatus extends longitudinally, transversally and vertically, the deflecting portion and the upper wall of the housing defining the discharging conduit extend in the parallel planes, said planes being defined by the longitudinal and transversal directions.

According to an embodiment, the discharge pipe and the upper wall of the housing defining the discharging conduit define a second angle, said second angle being comprised between 0° and 90°, preferably between 10° and 60°.

According to an embodiment, the discharge pipe is arranged within the discharging conduit in a way that the distance between the distal end of the deflecting portion and the upper wall of the housing with respect to the vertical direction is comprised between 20 and 80 mm and/or the shortest distance between the distal end of the deflecting portion and the outer surface of the forming drum, preferably the outer surface of the forming pockets with respect to the longitudinal direction is comprised between 100 and 1200 mm.

According to an embodiment, the apparatus comprises a discharge pipe orientation device enabling to change the inclination of the discharge pipe.

The present disclosure also relates to a method for forming moulded absorbent material deposit structures to be used as absorbent cores for absorbent articles using an apparatus as described hereabove, the method comprising:

- a defibrating step wherein a continuous sheet of absorbent material is defibrated by a defibrating machine at a defibrating station, thereby obtaining a plurality of absorbent fibers;
- a transport step wherein said plurality of absorbent fibers are conveyed by a second airflow flowing within the discharging conduit from the defibrating station to the forming pocket;
- a feeding step wherein a specific amount of superabsorbent particles in conjunction with the first airflow is sprayed from the discharge pipe, into the discharging conduit via the outlet nozzle;
- an accumulation step wherein the absorbent fibers and the superabsorbent particles accumulate within the mould cavity of the forming pocket thereby forming moulded absorbent material deposit structures to be used as absorbent cores for absorbent articles.

## BRIEF DESCRIPTION OF THE DRAWINGS

The drawings and figures are illustrative in nature and not intended to limit the subject matter defined by the claims. The following detailed description can be understood when read in conjunction with the following drawings, where similar or identical structures are indicated with the same reference numerals in which:

FIG. 1 illustrates a schematic side-view, or longitudinal cross-section, of an apparatus suitable for forming moulded absorbent material deposit structures to be used as absorbent cores for absorbent articles;

FIG. 2 shows a perspective view of a portion of the apparatus of FIG. 1, namely a perspective view of the portions of the discharging pipe feeding the superabsorbent particles and the duct conveying the absorbent fibers;

FIG. 3 depicts a schematic side-view, or longitudinal cross-section, of a portion of the apparatus of FIG. 1, namely a schematic side-view of the portions of the discharging pipe feeding the superabsorbent particles and the duct conveying the absorbent fibers;

FIG. 4 represents the same object and view as in FIG. 3 according to another embodiment as described herein;

FIG. 5 illustrates a schematic side-view, or longitudinal cross-section, of a portion of the apparatus of FIG. 1, namely a schematic side-view of the portions of the discharging pipe feeding the superabsorbent particles and the duct conveying the absorbent fibers ;

FIG. 6 shows a schematic cross section of a portion of the discharge pipe according to an embodiment as described herein ;

FIG. 7 depicts a schematic cross section of the outlet of the discharge pipe illustrating the principle of the invention, without being bound by theory, according to an embodiment as described herein,

FIG. 8 represents a schematic side-view, or longitudinal cross-section, of an apparatus suitable for forming moulded absorbent material deposit structures to be used as absorbent cores for absorbent articles according to another embodiment as described herein.

FIG. 9 illustrates a perspective view of a forming pocket.

## DETAILED DESCRIPTION

As discussed hereabove, there remains a need for a more efficient way of producing absorbent cores comprising a homogeneous, or uniform, distribution of superabsorbent particles. The present disclosure relates to an apparatus suitable for distributing superabsorbent particles uniformly onto a substrate and as well as an apparatus suitable for forming moulded absorbent material deposit structures to be used as absorbent cores for absorbent articles comprising said apparatus suitable for distributing superabsorbent particles homogeneously onto a substrate. The present disclosure also relates to a method for forming moulded absorbent material deposit structures to be used as absorbent cores using said apparatus. However, other mixtures of materials may be produced employing the present invention, depending on the particular parameters desired in the absorbent body. Such alternative configurations and uses are contemplated as being within the scope of the present invention.

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural unless the context clearly dictates otherwise. By way of example, "a compartment" refers to one or more compartments.

"About" as used herein referring to a measurable value such as a parameter, an amount, a temporal duration, and the like, encompass variations of +/-5% or less, even more preferably +/-1 % or less, and still more preferably +/-0.1% or less of and from the specified value, in so far such variations are appropriate to perform in the disclosed invention. However, it is to be understood that the value to which the modifier "about" refers is itself also specifically disclosed.

The term "channel(s)" as used herein means fluid distribution means within the absorbent core adapted to favor exudate flow there along and are typically intended to exclude embossing patterns or ducts formed by compression from the meaning thereof and rather include structures that are substantially free of absorbent material instead of comprising compacted absorbent material. Channels are commonly known in the art. Channels herein are formed by joining upper and lower layers of a core wrap as will be described in more detail hereinbelow. Channels preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width generally greater than 1 mm, preferably from 5 mm to 50 mm, more preferably from 6 mm to 40 mm, more preferably from 8 mm to 30 mm, even more preferably from greater than 8 mm to less than 25 mm. The one or more channels are arranged within the inner periphery of the absorbent core, i.e. the inner, or interior, space defined by the periphery of the absorbent core, such that each of the channel(s) is bounded, or surrounded, by absorbent material. Additionally, the length of the channels may be from 10 % to 95 % of the length of the absorbent core so that again the one or more channels are bounded by absorbent material.

"Comprise", "comprising", "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants and diaper holders and liners, Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. The absorbent article includes a chassis and may also include other components, such as liquid wicking layers, liquid intake layers, liquid distribution layers, transfer layers, barrier layers, wrapping layers and the like, as well as combinations thereof. Disposable absorbent articles and the components thereof can operate to provide a skin-facing surface and a garment-facing surface.

A "chassis" of a disposable absorbent article can include a liquid pervious topsheet, a backsheet joined to the top sheet, and an absorbent body positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The chassis comprises a front waist portion, a back waist portion, and an intermediate crotch portion which interconnects the front and back waist portions. When used herein, reference to a "front" portion refers to that part of the chassis which is generally located on the front of a wearer when in use. Reference to the "back" portion refers to the portion of the chassis generally located at the back of the wearer when in use, and reference to the "crotch" portion refers to that portion which is generally located between the legs of a wearer when in use. The crotch portion is an area where repeated fluid surge typically occurs.

The "absorbent core" or "absorbent body" as used herein are synonymous and correspond to the absorbent structure disposed between the topsheet and the backsheet of the chassis and is capable of absorbing and retaining liquid body exudates. The size and the absorbent capacity of the absorbent body should be compatible with the size of the intended wearer and the liquid loading imparted by the intended use of the chassis. Further, the size and the absorbent capacity of the absorbent body can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent body. It can contain embossed channels, channels substantially free of material, channels with lower basis weight than the rest of the body, etc.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent body.

The term "adhesive" as used herein is intended to refer to any suitable hot melt, water or solvent borne adhesive that can be applied to a surface in the required pattern or network of adhesive areas. Accordingly, suitable adhesives include conventional hot melt adhesives, pressure-sensitive adhesives and reactive adhesives (i.e., polyurethane).

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "backsheet" refers to a material forming a liquid impervious cover of the chassis of the absorbent article. The back sheet prevents the exudates contained in the absorbent body from wetting articles such as bedsheets and overgarments which contact the disposable absorbent article. The back sheet may be a composite layer composed of multiple components assembled side-by-side or laminated.

The term "channel", "conduit", "duct", "pipe" as used herein are synonyms and mean a tube or a passageway for conveying a fluid e.g. air, said channel, conduit, duct or pipe being defined by a housing or wall(s) defining an internal volume in which said fluid flows through. Similarly, "convey", "transport", "guide" are synonymous verbs and are interchangeable and mean that are moved, directed or conducted from one point to another.

The term "deflected" as used herein means deviated, ricocheted, rebounded off a surface, that has changed direction after hitting something, that has an obstacle in its course and is forced to change trajectory, that is caused to change direction or that is turn aside from a straight course. Preferably, this term is used herein as a significant change of direction or trajectory. In opposition the term "undeflected" means that does not significantly change direction, that is not or slightly deviated.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

By "fluidically connected" as used herein in reference to two elements is meant that a fluid, e.g. air, can circulate between these two elements. For example, two fluidically connected ducts means that a fluid can flow from one duct to the other.

The terms "fluff pulp", "pulp fibers", "fluff", "pulp" and "absorbent fibers" as used herein are all synonymous and interchangeable and generally reference to an absorbent material, preferably made out of fibers such as cellulosic fibers or any hydrophilic and absorbing fibers.

As used herein, the term "impermeable" or "impervious" generally refers to articles, chassis and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

The terms "inlet", "outlet", "upstream" and "downstream" are with respect to the flow direction of a fluid, e.g. an airflow.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The use of the term "layer" can refer, but is not limited to, any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

As used herein, the terms "longitudinal", "transversal" and "vertical" are with respect to the apparatus suitable for forming moulded absorbent material deposit structures to be used as absorbent cores for absorbent articles in an assembled state or installed state, said apparatus comprising the apparatus suitable for distributing superabsorbent particles homogeneously onto a substrate. The term "in the direction" as used herein means along that direction, e.g. in the longitudinal direction means along the longitudinal direction.

The terms "mesh", "foraminous forming screen" or "mesh substrate" as used herein are synonymous and mean a screen, a net, a material made of a network of wire(s) or thread(s), an interlaced structure or a weblike pattern or construction, all that have small openings to let air pass through while retaining, or blocking other material such as absorbent material, nonwoven material, or in other words that is air permeable.

The term "nonwoven substrate/material/web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

The terms "outlet nozzle" and "outlet section" as used herein are synonymous and interchangeable and refer to the same element, i.e. the extremity of the discharge pipe, namely the extremity of the discharging pipe arranged within the discharging conduit and enabling a crossflow of sub-streams.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "section" as used herein means a segment, a part, a portion of, a division or a component of an element. For example a section of a pipe means a portion of that pipe.

Use of the term "substrate" includes, but is not limited to, woven or nonwoven webs, porous films, ink permeable films, paper, composite structures, or the like.

The term "superabsorbent particle(s)", "superabsorbent polymer(s)", "SAP" and "superabsorbent polymer particle(s)" are interchangeable and all refer to a superabsorbent material suitable for use in the present disclosure are known to those skilled in the art, and may be in any operative form, such as particulate form and mixtures thereof. Generally stated, the "superabsorbent particles" can be a water-swellable, generally water-insoluble, hydrogel-forming polymeric absorbent material, which is capable of absorbing at least about 15, suitably about 30, and possibly about 60 times or more its weight in physiological saline (e.g., saline with 0.9 wt. % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system and may optionally be employed in other components or portions of the absorbent article. The superabsorbent material may be included in the absorbent layer or other fluid storage layer of the absorbent article of the present invention in an amount up to 10% by weight. Typically, the superabsorbent material, when present, will be included in an amount of from about 30% to about 70% by weight, based on the total weight of the absorbent layer. In addition, "superabsorbent particles", "superabsorbent polymer particles" or "SAPs" refer to water-swellable, water-insoluble organic or inorganic materials capable, under the most favorable conditions, of absorbing at least about ((10)) times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between ((10))0 to 800 $\mu$m, preferably between 300 to 600 $\mu$m, more preferably between 400 to 500 $\mu$m.

The term "topsheet" refers to a liquid permeable material sheet forming the inner cover of the chassis of an absorbent article and which in use is placed in direct contact with the skin of the wearer. The topsheet is typically employed to help isolate the wearer's skin from liquids held in the absorbent structure. The top sheet can comprise a nonwoven material, e.g., spunbond, meltblown, carded, hydroentangled, wetlaid or any other type of nonwoven.

Embodiments according to the disclosure will now be described. It is understood that technical features described in one or more embodiments may be combined with one or more other embodiments without departing from the intention of the disclosure and without generalization therefrom.

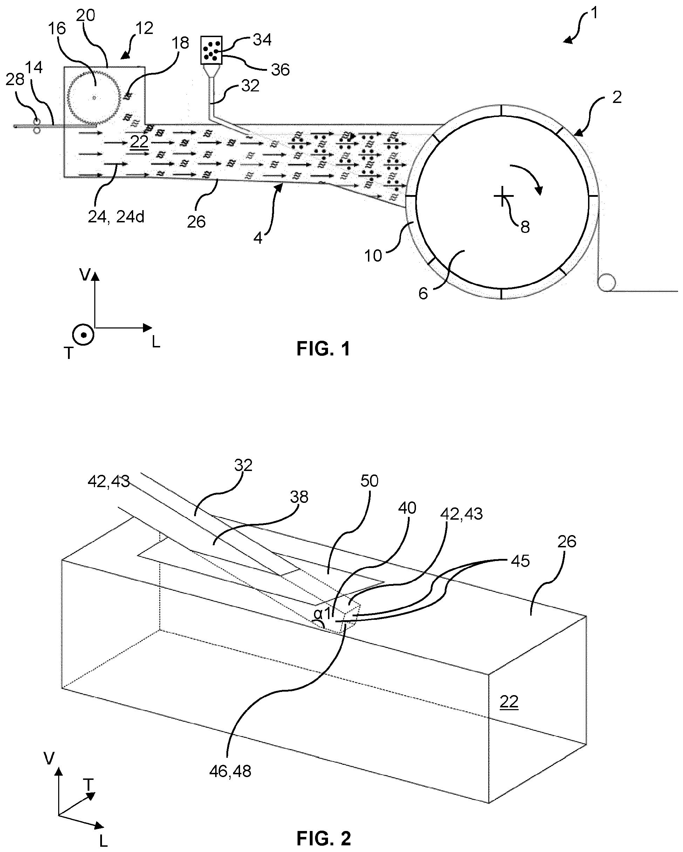

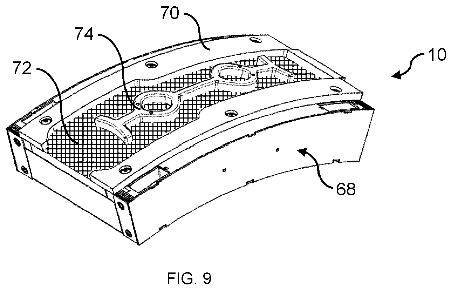

An apparatus 1 apparatus suitable for forming moulded absorbent material deposit structures to be used as absorbent cores for absorbent articles is illustrated in FIG. 1, or in other words, FIG. 1 represents a schematic longitudinal cross-sectional view of this apparatus 1 for manufacturing a continuous absorbent body. The apparatus 1 comprises a forming unit 2 and a feeding unit 4. The forming unit 2 comprises a rotary forming drum 6, also called a drum former, that rotates in a circumferential direction about an axis 8, and comprises an outer circumferential surface. A plurality of forming pockets 10 are arranged on the outer circumferential surface of the forming drum 6. Each forming pocket 10 defines a mould cavity where absorbent material is deposited therein thereby forming a moulded absorbent material deposit structure that can be used as an absorbent core for absorbent articles. Each forming pocket 10 comprises a mould cavity being bounded by at least one exterior surface, or by a masking component, defining the contour of the absorbent core to be formed and a bottom air permeable base surface. The bottom air permeable base surface comprises a foraminous forming screen, or mesh substrate, adapted to suck air while maintaining, or retaining, the absorbent material deposited from the feeding unit 4 thereon. The forming pocket 10 can optionally comprise a three-dimensional shaping profile member, or a channel insert, within the mould cavity to form a moulded absorbent material deposit structure, i.e. an absorbent core, with channels. FIG. 9 illustrates an embodiment of a forming pocket 10, comprising a base 68 or frame, supporting a mesh substrate, or foraminous forming screen 72, a masking component 70 defining the outer shape of the absorbent core and a channel insert 74. The foraminous forming screen 72, the masking component 70 and the channel insert 74 defining the mould cavity where the absorbent material is deposited. The absorbent body formed can be continuous or discontinuous depending on the forming pocket 10 used, on the absorbent material used, if the absorbent core is wrapped *etc.*

The absorbent material refers to the material constituting the absorbent core of the absorbent article. Examples of commonly occurring absorbent materials are selected from and not limited to cellulosic fluff pulp, tissue layers, highly absorbent polymers or superabsorbent polymer particles (SAP), absorbent foam materials, absorbent nonwoven materials or the like. For example, the absorbent core comprises cellulosic fluff pulp and/or superabsorbent particles, i.e. SAP.

The feeding unit 4 can comprise a defibrating station 12 that defibrates a continuous sheet of absorbent material 14 such as a sheet of cellulose material into fibers. The defibrating station 12 comprises: a defibrating machine 16 that defibrates the sheet of absorbent material thereby producing a plurality of absorbent fibers 18, or pulp fibers, or cellulosic fibers, fluff or fluff pulp and a defibration station housing 20 that covers the defibrating machine 16. The defibrating station 12 can also include a pair of feed rollers 28 that supplies the sheet of absorbent material 14 to the defibrating machine 16. The defibrating machine 16 is a device breaking the absorbent material sheet 14 into smaller pieces by grinding, crushing, or cutting, it can be for example a mill such as a hammermill.

The defibrating station 12 is connected, meaning fluidically connected, to a discharging conduit 22 through which a second airflow 24 flows (the first airflow will be mentioned hereunder), in a second airflow direction 24d. The second airflow 24 transports the absorbent fibers 18 produced in the defibrating station 12, in the discharging conduit 22. In other terms, the absorbent fibers 18 are conveyed by the second airflow 24 along the discharging conduit 22. The discharging conduit 22 corresponds to a channel defined by a housing 26 in which the second airflow 24 and the absorbent fibers 18 flow. In other terms, the housing 26 defines an internal or inner volume, i.e. the discharging conduit 22, where the second airflow 24 and absorbent fibers 18 circulate. The discharging conduit 22 fluidically connects the defibrating station 12 and the forming unit 2. In particular, the discharging conduit 22 comprises at least one inlet fluidically connected to the outlet of the defibrating station 12 and the discharging conduit 22 comprises at least one outlet fluidically connected to the inlet of the forming unit, meaning that one end of the discharging conduit 22 cover the outer peripheral surface of the forming drum 6.

The defibrating station 12, namely the defibrating machine 16 generates the second airflow 24. The defibrating machine 16 is a device which takes in a sheet of absorbent material 14 and shreds it into fibers with a rotating defibrating element 16. The defibrating machine 16, e.g. a hammermill, namely the rotating element, generates an air stream that flows within the discharging conduit 22 to the forming drum 6. In other words, the defibrating machine 16 generates the second airflow 24 with a volumetric flow rate ($m^3.s^{-1}$) comprised between 0,3 and 6 $m^3.s^{-1}$, preferably between 1 and 4 $m^3.s^{-1}$, depending on the rotation speed (RPM) of the hammermill that is comprised between 2000 and 5000 RPM (rotation per minute), preferably between 2500 and 3500 RPM.

The forming drum 6 is connected to a suction fan (not shown) through a suction duct (not shown). The suction fan is driven so that the pressure at a prescribed portion inside the forming drum 6 is kept negative. A substrate such as a now-woven web, e.g. a core wrap, or absorbent material can be applied on the mesh substrate, or foraminous forming screen, and be maintained on said foraminous forming screen with the negative pressure. The absorbent material such as SAP or fluff is deposited onto said non-woven substrate thereby forming a congregate of absorbent material suitable to be used as an absorbent core for an absorbent article. The absorbent material is maintained on the substrate and/or foraminous forming screen by the negative air-pressure generated by the suction, the pores formed in the mesh substrate functioning, or serving or acting, as suction holes. Moreover, the intake of air from the pores partially generates the second airflow 24. In other words, the suction fan generates a portion of the second airflow 24 within the discharging conduit 22, the pulp fibers 18 generated by the defibrating machine 16 are dispersed within the second airflow 24 and are guided to the forming drum 6. The outlet of the discharging conduit 22 is arranged in such a way that it covers at least a portion of the outer circumferential surface of the forming drum 6 and the forming pockets 10 arranged onto the outer circumferential surface of the forming drum 6. The suction fan further promotes the circulation of the second airflow 24. Hence, the absorbent fibers 18 are guided by the second airflow 24 from the defibrating station 12 to the mould cavity arranged within the forming pocket 10. As explained hereabove, the mould cavity comprises a mesh substrate so that the absorbent fibers are retained within the mould cavity to form a moulded absorbent material deposit structure, i.e. an absorbent core, the mesh substrate comprising micro-pores to let air pass through while retaining bigger objects such as fibers or super absorbent particles or a non-woven web.

As explained, hereabove, the absorbent material can also comprise superabsorbent particles (SAP). The absorbent core can comprise a combination of cellulosic fibers, i.e. fluff and superabsorbent particles. The absorbent core can also be essentially fluff-less meaning that the absorbent core contains less than 20% by weight fluff pulp, for example less than 10% fluff pulp, less than 5% fluff pulp or even no fluff pulp, or no more than an immaterial amount of fluff pulp which do not materially affect the thinness, flexibility or absorbency thereof.

As illustrated in FIG. 1, the feeding unit 4 of the apparatus 1 for forming moulded absorbent material deposit structures to be used as absorbent cores for absorbent articles comprises an apparatus suitable for distributing superabsorbent particles homogeneously onto a substrate, this substrate being the mould cavity of a forming pocket eventually already comprising a non-woven fabric arranged on the foraminous forming screen of the forming pocket 10. The apparatus suitable for distributing superabsorbent particles homogeneously onto a substrate comprises a superabsorbent particle discharge pipe 32 that supplies superabsorbent particles 34 into the discharging conduit 22. More particularly, the discharge pipe 32 conveys a stream of superabsorbent particle 34 and a first airflow 62 to the discharging conduit 22. The discharging conduit 22 and the discharge pipe 32 are fluidically connected, with the discharge pipe 32 extending partially within the discharging conduit 22 and comprising an outlet arranged within inner volume of the discharging conduit 22.

As illustrated in FIG. 1, the apparatus 1 for forming moulded absorbent material deposit structures extends in a longitudinal direction L (length), in a transversal direction T (width) and in a vertical direction V (height).

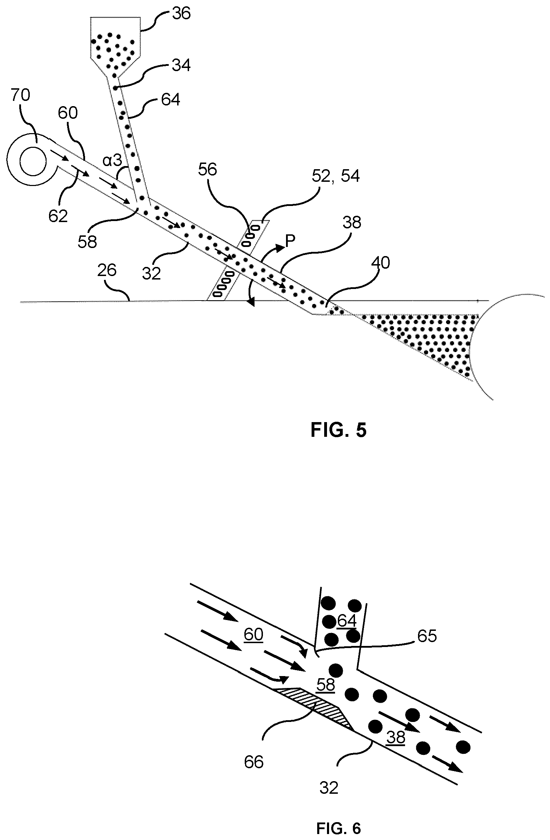

The discharge pipe 32 supplying, or discharging, superabsorbent particles 34 can be arranged in the upper part of the discharging conduit 22 or alternatively in the lower part of the discharging conduit 22 with respect to the vertical direction V. This discharge pipe 32 in conjunction with a first airflow 62 (FIG. 5), which has, or flows in, a first direction, supplies the SAP 34 and air into the discharging conduit 22. In other words, the superabsorbent particles 34 guided, or transported, in the discharge pipe 32 are dispersed within the discharging conduit 22 and are guided to the forming drum 6. The discharge pipe 32 channels, or conducts, a stream 35 comprising superabsorbent particles 34 and a first airflow 62. The outlet of the discharging conduit 22 is arranged in such a way that it covers at least a portion of the outer circumferential surface of the forming drum 6 and the forming pockets 10 arranged onto the outer circumferential surface of the forming drum 6. Hence, the superabsorbent particles 34 are guided by the first and second airflow 24 from a reservoir 36 such as a bag or container to the mould cavity arranged within the forming pocket 10. As explained hereabove, the mould cavity comprises a mesh substrate so that the superabsorbent particles 34 and/or absorbent fibers 18 are retained within the mould cavity to form a moulded absorbent material deposit structure, i.e. an absorbent core, the mesh substrate comprising micro-pores to let air pass through while retaining bigger objects such as fibers and/or super absorbent particles.

FIG. 2 illustrates a portion of the discharging conduit 22 defined by the housing 26 and a portion of the discharge pipe 32 which partially extends within the discharging conduit 22. The discharging conduit 22 comprises here a transversal cross section that is rectangular. The discharge pipe 32 comprises a conveying section 38 and an outlet section 40 comprising an outlet nozzle 40. The outlet nozzle 40 comprises a straight portion 42 arranged in the continuity of the conveying section 38 and a deflecting portion 46. The deflecting portion 46 comprises deflecting means 48 arranged in a way that only a portion of the stream of superabsorbent particles 34 and first airflow 62 are deflected and the other portion of said stream of superabsorbent particles 34 and first airflow 62 is not deflected by the outlet nozzle 40. In other words, the outlet nozzle 40 comprises a convergent portion 46 and a flat portion 42, the flat portion 42 being neither divergent nor convergent.

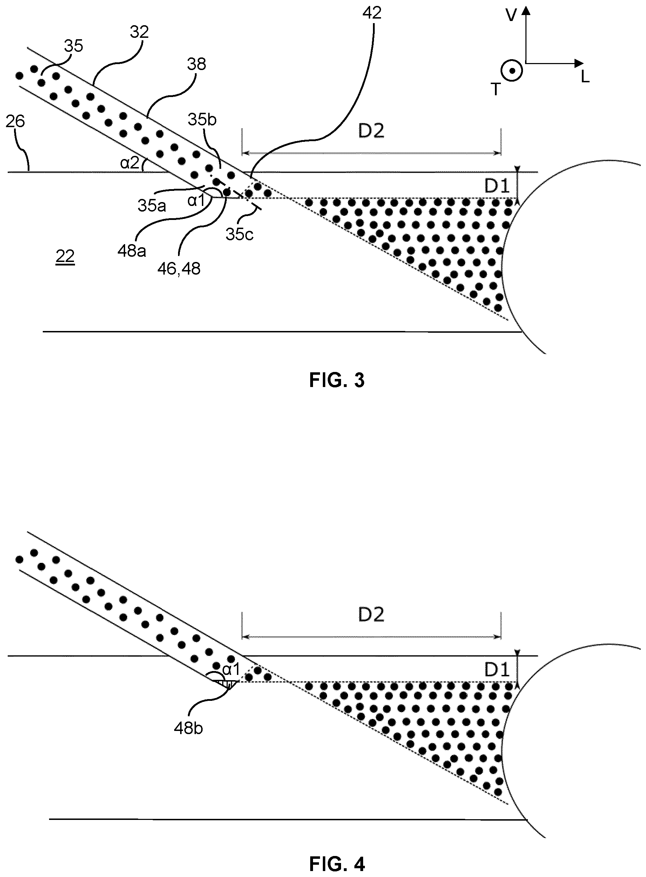

For sake of clarity, it is to be understood that the discharge pipe 32, in particular the conveying section 38, extends in a longitudinal direction L (length), in a transversal direction T (width) and in a vertical direction V (height), the outlet nozzle 40 is arranged in such a way that the straight portion 42 extends in the same directions (L,T,V) as the conveying section 38 whereas the deflecting portion 46 extends in at one in one direction that is not rectilinear to the directions in which the conveying section 38 extends in. In other words, the straight portion 42 and the conveying section 38 extend in a same plane whereas the deflection portion 46 and the conveying section 38 extend in different planes. According to the embodiment as illustrated in FIG. 3, the deflecting portion 46 does not extend in the vertical direction or at least does not extend in the same vertical direction as the conveying section 38, or in other words, the deflecting portion 46 extends only in a plane defined by the longitudinal and transversal directions unlike the conveying section.

As explained hereabove, the discharge pipe 32 conveys a stream 35 comprising superabsorbent particles 34 and a first airflow 62. As illustrated in FIG. 3, with such arrangement, the discharge pipe 32, and specifically the outlet nozzle 40 comprises deflecting means 48 arranged in a way that only a portion 35a of said stream 35 is deflected by the deflecting portion 46 and deflecting means 48 and the other portion 35b of said stream 35 is not deflected, meaning not deflected by the deflecting portion 46 and deflecting means 48. Such arrangement enables to create a crossflow between the portion 35a of the stream 35 that is deflected and the other portion 35b of the stream 35 that is not deflected, an imaginary delimitation 35c between the two portions 35a,35b being represented in FIG. 3 for illustrative purposes.

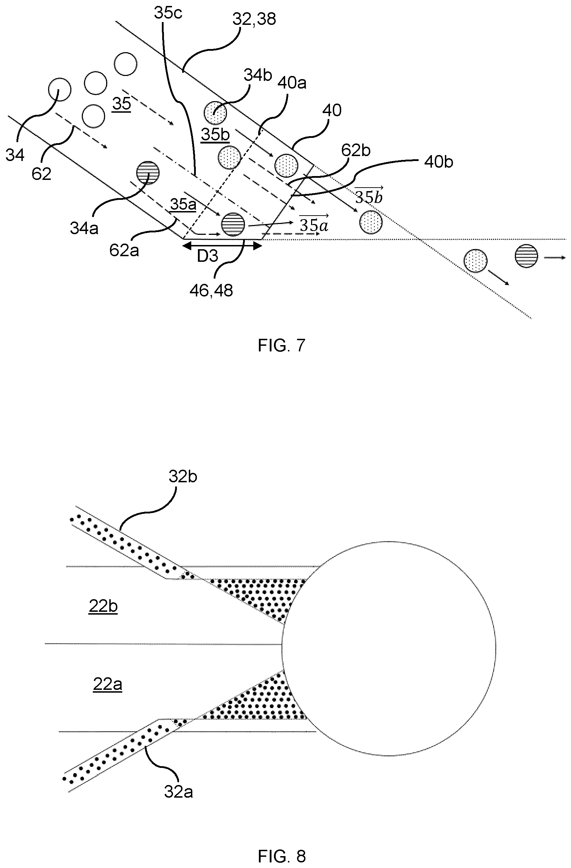

The inventors have observed that, surprisingly, such arrangement enables a uniform distribution of the superabsorbent particles 34 throughout the mould cavity arranged in the forming pocket 10. Without being bound by theory, it is believed that having the two portions 35a,35b, or two sub-streams 35a,35b, crossing generates a deviation of the superabsorbent particles 34 of both first deflected sub-stream 35a and second undeflected sub-stream 35b. This principle is illustrated in FIG. 7, in which the discharge pipe 32 and namely the conveying section 38 conveys, or transports or guides, a stream 35 comprising a first airflow 62 and superabsorbent particles 34. This stream 35 reaching the outlet section, meaning the outlet nozzle 40 is divided into two portions, or two sub-streams, 35a,35b : a first portion, or a first sub-stream 35a, comprising a portion of superabsorbent particles 34a, here represented in strips, and a portion of the first airflow 62a and a second portion, or a second sub-stream 35b, comprising a portion of superabsorbent particles 34b, here represented with dots, and a portion of the first airflow 62b. The first portion 35a is deflected by the deflecting means 48, meaning that the superabsorbent particles 34a and first airflow 62a of that first portion 35a have a change of direction or trajectory, e.g. a rebound or a curvature, and exit the discharge pipe 32 in a different direction than the second portion 35b. The second portion 35b is not deflected by the deflecting means 48, meaning that the superabsorbent particles 34b and first airflow 62b of that second portion 35b have a laminar flow and go in the same direction as the stream 35 in the conveying section 38 as illustrated in FIG. 7. There is thus a crossflow between the first portion 35a and the second portion 35b at the outlet of the discharge pipe 32.

Again without being bound by theory, the first airflow 62 comprising a greater volumetric flow rate than the superabsorbent particles 34 stream, it is believed that the first airflow 62a of the first portion 35a will deviate the course of the superabsorbent particles 34b of the second portion 35b and the first airflow 62b of the second portion 35b will deviate the course of the superabsorbent particles 34a of the first portion 35a leading to the superabsorbent particles 34 having the directions as illustrated in FIG. 7 and thereby leading to a uniform distribution as illustrated in FIG. 3.

It is to be understood that there is a first stream 35 of superabsorbent particles 34 and first airflow 62 flowing, or circulating, within the conveying section 38 in the same general direction(s) as the discharge pipe 32, meaning in the same longitudinal and vertical directions, in other words, the first stream 35 of superabsorbent particles 34 is guided, or conveyed, by the conveying section 38. The superabsorbent particles 34 in this first stream 35 can be slightly deflected by the walls of the discharge pipe 32 but generally flow in the general direction in which the discharge pipe extends in. The first stream 35 or superabsorbent particles 34 then reaches the outlet section, meaning the outlet nozzle 40, here the first stream 35 of superabsorbent particles 34 is figuratively divided in two sub-streams or portions, 35a,35b, a first sub-stream 35a, or a first portion 35a of the stream 35, where the superabsorbent particles 34 are deflected by the deflecting portion 46 comprising deflecting means 48 and a second sub-stream 35b, or a second portion 35b of the stream 35 where the superabsorbent particles 34 are not deflected by the deflecting portion 46. It is to be understood that the superabsorbent particles 34 of this first portion 35a are significantly deflected, in other words, the particles are deflected in such a way that they do not flow in the general direction of the discharge pipe 32 in particular when exiting the discharge pipe 32 through the outlet of said pipe 32. It is also to be understood that the superabsorbent particles 34 of this second portion 35b of the stream are not significantly deflected, in other words the superabsorbent particles 34 are not deflected or can be partially, or slightly, deflected by the flat, or straight, portion 42 in such a way that they flow in the general direction of the discharge pipe 32 in particular when leaving the discharge pipe 32 through the outlet of said pipe 32. In other words, the first portion 35a of the stream 35 has a substantially turbulent flow whereas the second portion 35b of the stream 35 has a substantially laminar flow.

It is also to be understood that by promoting a stream of absorbent particles 34 and first airflow 62 emerging from the discharge pipe 32 with a portion 35a that is deflected and a portion 35b that is undeflected, the outlet nozzle 40 enables a crossflow of these two portions of stream. This means that these two portions 35a,35b of stream 35, or sub-stream, will intersect, or in other words, there is a point of intersection between the flow direction of the first portion 35a that is deflected and the flow direction of the second portion 35b that is undeflected. Another way to illustrate this, is to represent the flow direction of a portion, or sub-stream, emerging from the outlet nozzle 40 (into the discharging conduit 22) by a corresponding vector $\overrightarrow{35a},\overrightarrow{35b}$. With this representation, it can be said that the outlet nozzle 40 enables to have a point of intersection between both vectors.

The deflecting means 48 comprise any mean for deflecting the superabsorbent polymer particles 34 and first airflow 62 flowing within the discharge pipe 32. For example, the discharge pipe 32 can comprise an elbow 48a, or a bent section, with a given angle or curvature, as illustrated in FIG. 3, or it can comprise a ramp or a plate 48b as illustrated in FIG. 4. In other terms, the deflecting means 48 comprise an element or a component that is either integral of, meaning constitutive of, the discharge pipe 32 or that is arranged within or fixed to the discharge pipe 32. Said deflecting means 48 control the flow, speed, direction, mass, shape, and/or the pressure of the stream 35 flowing and emerging from the discharge pipe 32. More specifically, the deflecting means 48 control the direction and change the trajectory of the superabsorbent particles 34 and first airflow 62 flowing within the discharge pipe 32 and emerging from said pipe 32, namely by partially, or locally, reducing the cross-sectional area of the pipe at the outlet of the discharge pipe 32. The deflecting means 48, for example the elbow 48a, define a first angle $\alpha1$ between the outlet nozzle 40 and the conveying section 38 as illustrated in FIG. 2 or FIG. 3. The first angle $\alpha1$ is obtuse, meaning that is it greater than 90° and less than 180°, preferably the first angle $\alpha1$ is comprised between 91° and 179°, preferably between 120° and 175°, more preferably between 150° and 170°, for example 162.5°. Similarly, the ramp 48b also defines a first angle $\alpha1$ as described herein between the outlet nozzle 40 and deflection portion 46 and the conveying section 38 as illustrated in FIG. 4.

As illustrated in FIG. 7, the outlet section, meaning the outlet nozzle 40 comprises an inlet 40a, meaning a proximal end, which is coupled, or linked or forms a continuity of matter with, to the conveying section 38 of the discharge pipe 32, and an outlet 40b meaning a distal end and also the outlet of the discharge pipe 32, which is arranged inside the discharging conduit 22. The outlet 40b is the frontier between the inner volumes of the discharge pipe 32 and the discharging conduit 22. The outlet nozzle 40 has a rectangular cross-sectional area along its length and comprises a top wall 43 corresponding to the straight or flat portion 42, a bottom wall corresponding to the deflecting portion 46 or deflecting means 48 and a first and second side wall 45. In other words, the outlet nozzle comprises a rectangular transversal cross section and the cross-sectional area of outlet nozzle is lesser than the cross-sectional area of the conveying section. For example the cross-sectional area of the conveying section is 3600 mm$^2$ whereas the cross-sectional area of the outlet nozzle 40 at the distal end is 2700 mm$^2$, or in other words, the cross-sectional area for the passage of the first airflow 62 and superabsorbent particles 34 is reduced by between 10 % and 40%, preferably by between 15% and 30%, for example by 25%. Proximal and distal are used herein in reference to the conveying section 38 of the discharge pipe 32, proximal being the point of the outlet nozzle 40 closest to the conveying section 38 and distal being the point of the outlet nozzle 40 farthest from the conveying section 38. The top wall 43, or straight portion 42, comprises a proximal end and a distal end. Both proximal and distal ends of the straight portion 42 are aligned with the conveying section 38 meaning that these ends are rectilinear with any point of the conveying section 38, meaning defining an angle of 180°. The bottom wall, or deflecting portion 46, comprises a proximal and a distal end. The distal end of the deflection portion 46 is unaligned with the bottom wall of the conveying section 38, or in other words, any point of the conveying section, the proximal end and the distal end of the deflection portion 46 are not aligned, meaning non-rectilinear thereby defining the angle $\alpha 1$.

Preferably, the distal end of straight portion 42 extends beyond the distal end of the deflecting portion 46 with respect to the longitudinal direction L as illustrated in FIG. 3 or 4. As illustrated in FIG. 7, the distal end and the proximal end of the deflecting portion 46 define a distance D3, D3 is preferably comprised between 25 mm and 70 mm, preferably between 30 and 60 mm, for example 43.5 mm. Similarly, the distance between the proximal and distal ends of the deflecting portion 46 is preferably greater than the distance between the proximal and distal ends of the straight portion 42. The inventors have observed that such arrangement leads to an good distribution of the superabsorbent particles 34 within the mould cavity.

The deflecting means 48 are arranged in a way that only a portion of the stream of superabsorbent particle 34 and first airflow 62 is deflected thereby creating a crossflow between the portion 35a of the stream that is deflected and the portion 35b of the stream that is not deflected by these deflecting means 48. It is thus possible to have an absorbent core where the superabsorbent polymer particles 34 are well, i.e. uniformly, distributed across the absorbent core, in particular across the length of the absorbent article.

The feeding unit 4 is designed so that the SAP 34 supplied by the discharge pipe 32 circulate in the discharging conduit 22. The discharge pipe 32 extends along the longitudinal and vertical direction L, V with the conveying section 38 extending inside and outside of the discharging conduit 22, the outlet nozzle 40 being arranged within the discharging conduit 22. The discharge pipe 32 and the housing 26 of the discharging conduit 22 define a second angle $\alpha 2$ as illustrated in FIG. 3, more precisely, the lower wall of the discharge pipe 32 and the upper wall of the housing 26 define said second angle $\alpha 2$. The second angle $\alpha 2$ is acute, meaning that it is comprised between 0° and 90°, preferably the second angle is comprised between 5° and 80°, preferably between 10° and 60°, for example 20°.

According to an embodiment as illustrated in FIG. 3, the deflecting means 48, meaning the lower wall of the outlet nozzle 40 extends in a direction, or a plane, substantially parallel to the direction, or plane, of the upper wall of the housing 26. The lower wall of the discharge pipe 32, namely of the conveying section 38 defining both angles $\alpha 1$ and $\alpha 2$, the feeding device 4 can comprise a discharge pipe 32 arranged in a way where the following equation (1) is respected:

$$\alpha 1 + \alpha 2 = 180° \ (1).$$

In other words, in an embodiment where the deflection portion 46 of the outlet nozzle 40 and the upper wall of the housing 26 extend in parallel planes defined by the longitudinal and transversal directions L, T, a wall, i.e. the lower wall of the conveying section 38, intersecting both planes, will respect equation (1). In other terms, the angles $\alpha 1$ and $\alpha 2$ can be complementary.

The feeding unit 4 can optionally comprise sealing means 50 at the junction between the discharge pipe 32 and the housing 26 as illustrated in FIG. 3. The sealing means 50 comprise for example a perforated foam or rubber plate in which the discharge pipe 32 goes through, said plate then being associated, or joined, with the housing 26. The sealing means 50 can also comprise a metal plate with a through-hole in which the discharge pipe is inserted that is welded onto the housing 26 and/or the discharge pipe 32. The sealing means 50 can also comprise adjustable stainless-steel plates closing the gap between the housing 26 and the discharge pipe 32.

According to an embodiment as illustrated in FIG. 5, the feeding unit 4 can also comprise a discharge pipe orientation device 52 that enables to change the inclination of the discharge pipe 32, or in other words enables to change the value of the second angle $\alpha 2$ between the discharge pipe 32 and the housing 26. In other words, the feeding unit 4 comprises means to pivot the discharge pipe 32 following a pivot direction P. The discharge pipe 32 can comprise at least one flexible portion, such as a corrugated plastic pipe connecting two metal pipes, to ease said pivoting. The pipe orientation device 52 here comprises a guide 54 for example a plate with slots 56 extending longitudinally that are either connected altogether through a slot extending vertically or isolated, the discharge pipe 32 comprising a pin to be lodged in said slots 56, e.g. a bayonet mount, the pipe orientation device 52 can also comprise an actuator (not illustrated) configured to move, preferably induce a movement of rotation and/or translation, the discharge pipe 32, the pipe 32 can also be moved manually. Hence with this device 52, it is possible to adjust the second angle ($\alpha 2$) of the discharge pipe 32 are readjust, or reposition, the outlet nozzle 40 to have a proper distribution of the superabsorbent particles within the forming pocket 10. The pipe orientation device 52 can also comprise a visual indicator on the guide 54 such as a scale with angles annotated thereby indicating the current value of the second angle $\alpha 2$, the plate with slots 56 can be linear or it can be curved or arcuate.

As illustrated in FIG. 5, the superabsorbent polymer discharge pipe 32 of the feeding unit 4 comprises an outlet section 40, meaning an outlet nozzle 40, a conveying section 38 as described hereabove, and a venturi section 58. In particular, the discharge pipe 32 comprises a first feeding tube 60 in which a first airflow 62 is flowing, meaning is conveyed. The discharge pipe 32 comprises a second feeding tube 64 linked to the SAP reservoir 36 in which superabsorbent particles 34 are flowing, meaning are conveyed. The first and second feeding tubes 60,64 are both fluidically connected to the conveying section 38. The second feeding tube 64, meaning the tube feeding or supplying superabsorbent particles 34 to the conveying section 38, penetrates within the first feeding tube 60 thereby reducing the cross-sectional area for the passage of air for the first airflow 62 and thereby creating a venturi effect. Specifically, the second feeding tube 64 by entering the first feeding tube 60 and extending partially within said first feeding tube 60 creates a constriction in said first feeding tube 60 and reduces the air pressure thereby increasing the fluid speed of the first airflow 62 past that constriction point. The superabsorbent polymer particles 34 are conveyed through the second feeding tube 64 mostly via gravity, and then in the conveying section 38 via the first airflow 62.

According to an embodiment, the forming unit 4 comprises an air-generating device 70 able to move air, namely able to blow air within the first feeding tube 60, the first feeding tube 60 comprising an inlet fluidically connected to an air-generating device 70. The air-generating device 70 is for example a blower or a centrifugal fan as illustrated in FIG.5, but other means of moving air are possible such as an axial fan or more generally any air pump. The air-generating device 70 can comprise a housing in which is arranged an impeller moved by a driving mechanism such as a shaft actuated by an electric motor. Said air-generating device 70 generates the first airflow 62 with a given volumetric flow rate $(m^3.s^{-1})$.

The first and second feeding tube 60,64, define a third angle $\alpha 3$, particularly, the lower wall of the second feeding tube 64 and the upper wall of the first feeding tube 60 define this third angle $\alpha 3$. The third angle $\alpha 3$ is preferably acute, meaning that it is comprised between 0° and 90°, preferably the third angle is comprised between 10° and 80°, preferably between 15° and 60°, for example 40°. Such arrangement enables the superabsorbent particles 34 to be introduced in the conveying section 38 and conveyed by the first airflow 62 to the outlet nozzle 40 with less turbulence. If the third angle $\alpha 3$ is too low, meaning less than 5°, the SAP will not fall down properly, the SAP will have too much friction with the second feeding pipe 64 and gravity cannot ensure a proper conveying of the particles 34, alternatively, if the third angle $\alpha 3$ is greater

than 90°, the SAP will end up in the conveying section 38 abruptly thereby generating significant turbulences in the stream 35.

According to an embodiment as illustrated in FIG. 6, the venturi section 58 can comprise an obstacle 66 that is arranged in discharge pipe 32, i.e. within the internal volume of said pipe 32, at the junction between the first feeding tube 60, the second feeding tube 64 and the conveying section 38. The obstacle 66 can be a plate, rib, bloc or any mean that can deviate the first airflow 62 and reduce the cross-sectional area of the discharge pipe 32 thereby generating a pressure drop and increasing the volumetric flow rate of the first airflow 62. Additionally, the second feeding tube 64 can optionally comprise a wall 65 partially extending within the first feeding tube 60 to further reduce cross-sectional area of the discharge pipe 32 and increase the volumetric flow rate of the first airflow 62. Said wall 65 can be curved as illustrated in FIG. 6 or flat (for example as illustrated in FIG. 5).

According to an embodiment, the gap defined by obstacle 66 and/or wall 65 defines a gap comprised between 1 and 10 mm, preferably between 3 and 7 mm, for example with respect to the height of the cross-sectional area of the discharging pipe 32 at the venturi section 58. With such gap, the air-generating device 70 preferably functions at a rotational speed, or frequency, comprised between 1500 and 2200 RPM, for example 1800 RPM thereby generating a first airflow 62 with a speed comprised between 28 to 35 $m.s^{-1}$ at the inlet of the venturi section 38. The inventors have observed that these parameters lead to an good distribution of the absorbent material.

According to an embodiment, the discharge pipe 32 is arranged within the discharging conduit 22 so that the distal end of the deflecting portion 46 and the upper wall of the housing 26 defines a distance D1 with respect to the vertical direction, D1 being comprised between 25 and 80 mm. Similarly, the discharge pipe 32 is arranged within the discharging conduit 22 so that the distal end of the deflecting portion 46 and the outer surface of the forming drum 6 meaning the forming pocket 10, defines a minimal distance D2 with respect to the longitudinal direction, D2 being comprised between 100 and 1200, preferably between 200 and 1000 mm. The inventors have observed that these parameters lead to an good distribution of the absorbent material.

With respect to the direction of flow, the discharging pipe 32 comprises a first section comprising the first feeding tube 60 and the second feeding tube 64, a second section comprising the conveying section 38, a venturi section 58 being arranged in-between said first and second sections, and the outlet section or outlet nozzle. These first and second sections can be identical in shape and/or dimension. Alternatively, these first and second sections can be different in shape and/or dimension. For example, the first section can comprise a circular cross section whereas the second section comprises a rectangular cross-section. A transition is made between the circular cross section of the first section and the rectangular cross section of the second section by using curved surfaces that gradually change the shape of the cross section between circle and rectangle, passing continuously through of a plurality of intermediate cross sections of different shapes.

In another embodiment, the apparatus for manufacturing a continuous absorbent body comprises a first covering unit that supplies a first endless substrate precursor, preferably a nonwoven web, that covers the plurality of forming pockets 10 of the forming drum 1, before providing the homogeneous mixture of pulp fibers and SAP in the forming pockets 10. After placing the homogeneous mixture of absorbent fibers 18 and SAP 34 on the first endless substrate precursor, folding guide plates (not shown) fold both lateral sides of the first substrate precursor in the width direction to cover the mixture absorbent fibers 18 and SAP 34 thereby defining a continuous wrapped absorbent body.

According to an embodiment, instead of comprising folding guide plates, the apparatus for manufacturing a continuous absorbent body comprises a second covering unit that supplies a second endless substrate precursor, preferably a nonwoven web. Said second endless substrate precursor is disposed on the homogeneous mixture of absorbent fibers 18 and SAP 34 in such a way that said homogeneous mixture is arranged and enveloped between the first and second endless nonwoven substrates thereby defining a continuous wrapped absorbent body.

According to an embodiment as illustrated in FIG. 8, the absorbent core can comprise two layers of absorbent material 18,34. The feeding unit 4 comprises in this case two discharging conduits 22a,22b linked to the defibrating station 12 (not illustrated). The feeding unit 4 comprises two discharge pipes 32a,32b as described herein, each with an outlet nozzle 40 as described herein. It this possible to have an absorbent article comprising two layers of absorbent fibers 18 and/or superabsorbent particles 34. The two layers can be identical, or they can comprise different amounts of absorbent fibers 18 and/or superabsorbent particles 34. For example, if the lower discharge pipe 32a comprises a blower functioning at a lower rotational speed than the blower comprised in the upper discharge pipe 32b, e.g. 1500 RMP and 1800 RPM respectively, then the amount of SAP will be lesser in the lower layer of the absorbent core. According to another example, both discharge pipe 32a,32b exploit one air-generating device that is fluidically connected to both pipes, but the venturi section in said pipes is different, e.g. obstacle 66 of different dimensions, hence leading also to the layers of the absorbent core having different amount of SAP 34.

According to an embodiment not illustrated, one of the side walls 45 of the outlet nozzle 40 comprises deflecting means 48 as described herein. The deflecting means 48 are arranged in a way that only a portion of the stream of superabsorbent particle 34 and first airflow 62 is deflected thereby creating a crossflow between the portion 35a of the stream that is deflected and the portion 35b of the stream that is not deflected by these deflecting means 48. It is thus possible to have an absorbent core where the superabsorbent polymer particles 34 are well, i.e. uniformly, distributed across the absorbent core, in particular across the width of the absorbent article. In this embodiment, the side walls 45 both comprise a proximal end and a distal end in reference to the conveying section 38. One first side wall 45 comprises both proximal and distal ends aligned with the conveying section 38 meaning that these ends of this first side wall 45 are rectilinear with any point of the conveying section 38, meaning defining an angle of 180°. The distal end of the other second side wall 45 is unaligned, not aligned, with the side wall of the conveying section 38, or in other words, any point of the conveying section 38, the proximal end and the distal end of this other second side wall are not aligned, meaning non-rectilinear thereby defining the angle α1. Preferably, the distal end of first side wall 45 extends beyond the distal end of the other second side wall 45 with respect to the longitudinal direction L.

According to an embodiment not illustrated, one of the side walls 45 of the outlet nozzle comprises deflecting means 48 as described herein and the bottom wall 46 of the outlet nozzle comprises deflecting means 48 as described herein. It is thus possible to have an absorbent core where the superabsorbent polymer particles 34 are well, i.e. uniformly, distributed across the absorbent core, in particular across the length and width of the absorbent article.

As mentioned previously, the disclosure also pertains to an apparatus 1 suitable for forming moulded absorbent material deposit structures to be used as absorbent cores for absorbent articles, said deposit structures comprising a uniform or homogeneous distribution of superabsorbent particles 34, the apparatus 1 comprising:

- a forming unit 2 comprising: a forming drum 6 comprising an outer circumferential surface, a plurality of forming pockets 10 being arranged on the outer circumferential surface of said forming drum 6, each forming pocket 10 comprising a mould cavity; and

- a feeding unit 4 comprising:

    ◦ a defibrating station 12 configured to generate pulp fibers,
    ◦ a discharging conduit 22 defined by a housing 26 fluidically connecting the defibrating station 12 and the forming unit 2 through which the plurality of absorbent fibers 18 and a second airflow 24 flow,
    ◦ an apparatus for distributing superabsorbent particles 34 as described herein.

According to the present disclosure, the discharge pipe 32 extends partially within the discharging conduit 22, the outlet nozzle 40 being arranged within the discharging conduit 22 in a way that the discharge pipe 32 sprays superabsorbent particles 34 into the discharging conduit 22, and into the mould cavity of the forming pockets 10.

Incidentally, the outlet nozzle 40 feeds the superabsorbent particles 34 into the discharging conduit 22, creating a homogeneous mixture of absorbent fibers 18 and superabsorbent particles 34, said homogeneous mixture being deposited in the mould cavity, meaning on the foraminous forming screen, of the forming pockets 10 arranged on the forming drum 1.

According to an embodiment, the deflection portion 46 is oriented substantially parallel to the direction of the first airflow 24.

The method for manufacturing a continuous absorbent core using an apparatus as described herein. For sake of clarity said apparatus comprises: a forming unit 2 and a feeding unit 4; the forming unit 2 having: a forming drum 6 comprising an outer circumferential surface, a plurality of forming pocket 10 being arranged on the outer circumferential surface of the forming drum 1, said forming pocket 10 comprising a mould cavity defined by a foraminous forming screen and eventually a masking element disposed within the pockets 10; and the feeding unit 4 comprising a defibrating station 12 with a defibrating machine 16, such as a hammermill, a discharging conduit 22 through which a second airflow 24 flows, a superabsorbent polymer discharge pipe 32 through which a first airflow 62 flows comprising an outlet nozzle 40 as described herein. The method for manufacturing a continuous absorbent body comprises;

- a defibrating step wherein a continuous sheet of absorbent material 14 is supplied and defibrated by the defibrating machine 16 at the defibrating station 12, thereby obtaining a plurality of absorbent fibers 18;

- a transport step wherein said plurality of absorbent fibers 18 are transported, or conveyed or guided, by the second airflow 24 which generated by the defibrating machine 16 and the suction fan arranged in the forming unit 2, said fibers 18 are guided from the defibrator station 12, through the second airflow 24 circulating inside the discharging conduit 22 to the forming unit 2 and the forming pocket 10;

- a feeding step wherein a specific amount of superabsorbent particles 34 in conjunction with the first airflow 62 is sprayed through the superabsorbent polymer discharge pipe 32 and the outlet nozzle 40 into the discharging conduit 22 via the outlet nozzle 40, said outlet nozzle creating a crossflow of superabsorbent particles 34,

- eventually a mixing step, wherein the plurality of absorbent fibers 18 transported by the second airflow 24 are mixed with the crossflow of SAP sprayed by the discharge pipe 32 and the outlet nozzle 40; and

- an accumulation step wherein the absorbent fibers 18 and the superabsorbent particles 34 accumulate within the mould cavity of the forming pocket 10 thereby forming an absorbent core structure.

As written hereabove, according to an embodiment, it is possible to make fluff-less absorbent core, meaning that the hereabove method can only comprise the spraying of superabsorbent particles 34 within the mould cavity as described herein.

In an additional embodiment, the method can comprise an additional step, a covering step performed prior to the accumulation step, in which a covering substrate precursor, in other words a core wrap, such as a non-woven material, is applied to cover the mould cavity of forming pockets 10. Subsequently, the accumulation step is carried out where the absorbent material, meaning the absorbent fibers 18 and the superabsorbent particles 34, is deposited into the mould cavity and onto the covering substrate precursor. After the accumulation step, a folding step is carried out where the covering substrate precursor is folded over the mixture of pulp fibers and superabsorbent particles 34, thereby defining a wrapped absorbent core. Alternatively, instead of folding over the covering substrate precursor, the method can comprise a second covering step where a second covering substrate, in other words a second core wrap, such as a layer of non-woven material, is applied to cover the mixture of pulp fibers and superabsorbent particles 34 thereby defining a wrapped absorbent core.

As seen previously, the forming pocket 10 and mould cavity can comprise a channel insert, the method can comprise a further pressing step, where pressing means, such as one or more rollers, apply pressure onto the wrapped absorbent core to ensure that the core wraps are properly joined, or bonded, together at the periphery and/or at the central area thereby ensuring a proper sealing at the periphery and/or ensuring a proper formation of channel(s). Adhesive can be applied on the core warp(s) to strengthen the bonding.

The method can also comprise a removal step, where a surplus of absorbent material can be removed by removal means, such as a brush with bristles or a blower, from the mould cavity and/or channel insert to have a uniform amount of absorbent material in the absorbent cores, said removal step being after the accumulation step and before the covering step.

In an additional embodiment, namely if the absorbent core comprises two layers of absorbent material, the method can comprise as an accumulation step:

- a first covering step in which a first covering substrate precursor, meaning a bottom core wrap, such as a non-woven material, is applied to cover the mould cavity of forming pockets 10,

- a first accumulation step, in which the absorbent material, meaning the pulp fibers and superabsorbent particles 34, is deposited into the mould cavity and onto the first covering substrate precursor;

- a second covering step, in which a second covering substrate, meaning an intermediate core wrap such as a non-woven material, is applied to cover the absorbent material,

- a second accumulation step in which absorbent material is deposited into the mould cavity and onto the second covering substrate; and

- a third covering step in which a third covering substrate, meaning a top core-wrap such as a non-woven material, is applied to cover the absorbent material, thereby defining a wrapped absorbent core comprising two layers of absorbent material.

**Claims**

1. Apparatus for distributing superabsorbent particles (34) onto a substrate, the apparatus comprising a discharge pipe (32) for conveying a stream (35) comprising superabsorbent particles (34) and a first airflow (62), said discharge pipe (32) comprising a conveying section (38) and an outlet nozzle (40), said outlet nozzle (40) comprising a straight portion (42) and a deflecting portion (46) **characterized in that** the deflecting portion (46) comprises deflecting means (48) arranged in a way that only a portion (35a) of the stream (35) of superabsorbent particle (34) and first airflow (62) is deflected and the other portion (35b) of said stream is not deflected, creating a crossflow between the portion of the said stream (35a) that is deflected and the portion of said stream (35b) that is not deflected.

2. Apparatus for distributing superabsorbent particles (34) onto a substrate according to claim 1, wherein the deflecting means (48,48a,48b) define a first angle ($\alpha$1) between the conveying section (38) and the deflection portion (46), said first angle ($\alpha$1) being comprised between 91° and 179°, preferably between 120° and 175°.

3. Apparatus for distributing superabsorbent particles (34) onto a substrate according to any of the preceding claims, wherein the discharge pipe (32) extends longitudinally, transversally and vertically (L,T,V), said outlet nozzle (40) comprises a top wall (42), a bottom wall (46), a first and second side wall (45), each wall comprising a proximal end connected to the conveying section (38) and a distal end arranged at the outlet of the discharging pipe (32), the distal end of the top wall (42) extending longitudinally beyond the distal end of the bottom wall (46) and/or the distal end of the first side wall (45) extending longitudinally beyond the distal end of the second side wall (45).

4. Apparatus for distributing superabsorbent particles onto a substrate according to any of the preceding claims, wherein the discharge pipe (32) further comprises a first feeding tube (60) conveying a first airflow (62), a second feeding tube (64) conveying superabsorbent particles (34) and a venturi section (58) arranged at the junction between the first and second feeding tubes (60,64) and the conveying section (38), the first and second feeding tubes (60,64) and the conveying section (38) all being fluidically connected, first and second feeding tubes (60,64) being arranged upstream of the conveying section (38) with respect to the direction of flow.

5. Apparatus for distributing superabsorbent particles onto a substrate according to claim 4, wherein the venturi section (58) is formed by the second feeding tube (64) partially extending within first feeding tube (60) and reducing the cross-sectional area of the discharge pipe (32) for the first airflow (62).

6. Apparatus for distributing superabsorbent particles onto a substrate according to any of the claims 4 or 5, wherein the first feeding tube (60) and the second feeding tube (64) define a third angle ($\alpha$3), said third angle ($\alpha$3) being comprised between 0° and 90°, preferably between 15° and 60°.

7. Apparatus for distributing superabsorbent particles onto a substrate according to any of the preceding claims, wherein the outlet nozzle (40) comprises a rectangular transversal cross section and the cross-sectional area of outlet nozzle (40) is lesser than the cross-sectional area of the conveying section (38).

8. An apparatus (1) suitable for forming moulded absorbent material deposit structures to be used as absorbent cores for absorbent articles, the apparatus (1) comprising:

   - a forming unit (2) comprising: a forming drum (6) comprising an outer circumferential surface, a plurality of forming pockets (10) arranged on the outer circumferential surface of said forming drum (6), each forming pocket (10) comprising a mould cavity; and
   - a feeding unit (4) comprising:

      ○ a defibrating station (12) configured to generate absorbent fibers (18) and a second airflow (24),

◦ a discharging conduit (22) defined by a housing (26) fluidically connecting the defibrating station (12) and the forming unit (2) through which the plurality of absorbent fibers (18) and a second airflow (24) flow,

◦ an apparatus for distributing superabsorbent particles (34) according to claim 1 to 7,

wherein the discharge pipe (32) extends partially within the discharging conduit (22), the outlet nozzle (40) being arranged within the discharging conduit (22) in a way that the discharge pipe (32) sprays superabsorbent particles (34) into the discharging conduit (22) and into the mould cavity of the forming pockets (10).

9. Apparatus (1) suitable for forming moulded absorbent material deposit structures according to claim 8, wherein the feeding unit (4) comprises two discharging conduits (22a,22b), each conduit (22a,22b) being fluidically connected to the defibrating station (12) and each conduit (22a,22b) comprising a discharge pipe (32a,32b) extending partially within its respective discharging conduit (22a,22b), the outlet nozzle of each pipe (32a,32b) being arranged within its respective discharging conduit (22a,22b) .

10. Apparatus (1) suitable for forming moulded absorbent material deposit structures according to any of the claim 8 or 9, wherein the apparatus (1) extends longitudinally, transversally and vertically (L, T, V), the deflecting portion (46) and the upper wall of the housing (26) defining the discharging conduit (22) extend in the parallel planes, said planes being defined by the longitudinal and transversal directions.

11. Apparatus (1) suitable for forming moulded absorbent material deposit structures according to any of the claims 8 to 10, wherein the discharge pipe (32) and the upper wall of the housing (26) defining the discharging conduit (22) define a second angle ($\alpha$2), said second angle ($\alpha$2) being comprised between 0° and 90°, preferably between 10° and 60°.

12. Apparatus (1) suitable for forming moulded absorbent material deposit structures according to any of the claims 8 to 11, wherein the discharge pipe (32) is arranged within the discharging conduit (22) in a way that the distance (D1) between the distal end of the deflecting portion (46) and the upper wall of the housing (26) with respect to the vertical direction (V) is comprised between 20 and 80 mm and/or the shortest distance (D2) between the distal end of the deflecting portion (46) and the outer surface of the forming drum (6), preferably the outer surface of the forming pockets (10) with respect to the longitudinal direction (L) is comprised between 100 and 1200 mm.

13. Apparatus (1) suitable for forming moulded absorbent material deposit structures according to any of the claims 8 to 12, wherein the apparatus (1) comprises a discharge pipe orientation device (52) enabling to change the inclination of the discharge pipe (32).

14. A method for forming moulded absorbent material deposit structures to be used as absorbent cores for absorbent articles using an apparatus according to any of the claims 8 to 13, the method comprising:

a) a defibrating step wherein a continuous sheet of absorbent material (14) is defibrated by a defibrating machine (16) at a defibrating station (12), thereby obtaining a plurality of absorbent fibers (18);
b) a transport step wherein said plurality of absorbent fibers (18) are conveyed by a second airflow (24) flowing within the discharging conduit (22) from the defibrating station (12) to the forming pocket (10);
c) a feeding step wherein a specific amount of superabsorbent particles (34) in conjunction with the first airflow (62) is sprayed from the discharge pipe (32), into the discharging conduit (22) via the outlet nozzle (40);
d) an accumulation step wherein the absorbent fibers (18) and the superabsorbent particles (34) accumulate within the mould cavity of the forming pocket (10) thereby forming moulded absorbent material deposit structures to be used as absorbent cores for absorbent articles.

**Patentansprüche**

1. Einrichtung zum Verteilen von superabsorbierenden Partikeln (34) auf ein Substrat, die Einrichtung umfassend ein Auslassrohr (32) zum Fördern eines Stroms (35), umfassend superabsorbierende Partikel (34) und eine erste Luftströmung (62), das Auslassrohr (32) umfassend eine Fördersektion (38) und eine Ablaufdüse (40), die Ablaufdüse (40) umfassend einen geraden Abschnitt (42) und einen Umlenkabschnitt (46), **dadurch gekennzeichnet, dass** der Umlenkabschnitt (46) Umlenkmittel (48) umfasst, die auf eine Weise eingerichtet sind, dass nur ein Abschnitt (35a) des Stroms (35) aus superabsorbierenden Partikeln (34) und erster Luftströmung (62) umgelenkt wird und der andere Abschnitt (35b) des Stroms nicht umgelenkt wird, wodurch eine Querströmung zwischen dem Abschnitt des Stroms (35a), der umgelenkt ist, und dem Abschnitt des Stroms (35b) entsteht, der nicht umgelenkt ist.

2. Einrichtung zum Verteilen von superabsorbierenden Partikeln (34) auf ein Substrat nach Anspruch 1, wobei die Umlenkmittel (48,48a,48b) einen ersten Winkel (α1) zwischen der Fördersektion (38) und dem Umlenkabschnitt (46) definieren, wobei der erste Winkel (α1) zwischen 91° und 179°, vorzugsweise zwischen 120° und 175°, umfasst ist.

3. Einrichtung zum Verteilen von superabsorbierenden Partikeln (34) auf ein Substrat nach einem der vorstehenden Ansprüche, wobei das Auslassrohr (32) sich in Längsrichtung, Querrichtung und Vertikalrichtung (L, T, V) erstreckt, die Ablaufdüse (40) eine obere Wand (42), eine untere Wand (46), eine erste und eine zweite Seitenwand (45) umfasst, jede Wand umfassend ein proximales Ende, das mit der Fördersektion (38) verbunden ist, und ein distales Ende, das an dem Ablauf des Auslassungsrohrs (32) eingerichtet ist, wobei das distale Ende der oberen Wand (42) sich in Längsrichtung über das distale Ende der unteren Wand (46) hinaus erstreckt und/oder das distale Ende der ersten Seitenwand (45) sich in Längsrichtung über das distale Ende der zweiten Seitenwand (45) hinaus erstreckt.

4. Einrichtung zum Verteilen von superabsorbierenden Partikeln auf ein Substrat nach einem der vorstehenden Ansprüche, wobei das Auslassrohr (32) ferner ein erstes Zufuhrrohr (60), das eine erste Luftströmung (62) zuführt, ein zweites Zufuhrrohr (64), das superabsorbierende Partikel (34) zuführt, und eine Venturisektion (58) umfasst, die an der Verzweigung zwischen dem ersten und dem zweiten Zufuhrrohr (60,64) und der Fördersektion (38) eingerichtet ist, wobei das erste und das zweite Zufuhrrohr (60,64) und die Fördersektion (38) alle fluidisch verbunden sind, das erste und das zweite Zufuhrrohr (60,64) in Bezug auf die Strömungsrichtung stromaufwärts der Fördersektion (38) eingerichtet sind.

5. Einrichtung zum Verteilen von superabsorbierenden Partikeln auf ein Substrat nach Anspruch 4, wobei die Venturisektion (58) durch das zweite Zufuhrrohr (64) ausgebildet wird, das sich teilweise innerhalb des ersten Zufuhrrohrs (60) erstreckt und die Querschnittsfläche des Auslassrohrs (32) für die erste Luftströmung (62) verringert.

6. Einrichtung zum Verteilen von superabsorbierenden Partikeln auf ein Substrat nach einem der Ansprüche 4 oder 5, wobei das erste Zufuhrrohr (60) und das zweite Zufuhrrohr (64) einen dritten Winkel (α3) definieren, wobei der dritte Winkel (α3) zwischen 0° und 90°, vorzugsweise zwischen 15° und 60° umfasst ist.

7. Einrichtung zum Verteilen von superabsorbierenden Partikeln auf ein Substrat nach einem der vorstehenden Ansprüche, wobei die Ablaufdüse (40) einen rechteckigen transversalen Querschnitt umfasst und die Querschnittsfläche der Ablaufdüse (40) kleiner als die Querschnittsfläche der Fördersektion (38) ist.

8. Einrichtung (1), die zum Ausbilden geformter Ablagerungsstrukturen aus absorbierendem Material geeignet ist, die als absorbierende Kerne für absorbierende Artikel verwendet werden sollen, die Einrichtung (1) umfassend:

   - eine Ausbildungseinheit (2), umfassend: eine Ausbildungswalze (6), umfassend eine äußere Umfangsoberfläche, eine Vielzahl von Ausbildungstaschen (10), die auf der äußeren Umfangsoberfläche der Ausbildungswalze (6) eingerichtet sind, jede Ausbildungstasche (10) umfassend einen Formhohlraum; und
   - eine Zufuhreinheit (4), umfassend:

      ◦ eine Zerfaserungsstation (12), die konfiguriert ist, um absorbierende Fasern (18) und eine zweite Luftströmung (24) zu erzeugen,
      ◦ eine Auslassungsleitung (22), die durch ein Gehäuse (26) definiert ist, das die Zerfaserungsstation (12) und die Ausbildungseinheit (2) fluidisch verbindet, durch die die Vielzahl der absorbierenden Fasern (18) und eine zweite Luftströmung (24) strömen,
      ◦ eine Einrichtung zum Verteilen von superabsorbierenden Partikeln (34) nach Anspruch 1 bis 7,
      wobei das Auslassrohr (32) sich teilweise innerhalb der Auslassungsleitung (22) erstreckt, die Ablaufdüse (40) innerhalb der Auslassungsleitung (22) auf eine Weise eingerichtet ist, dass das Auslassrohr (32) superabsorbierende Partikel (34) in die Auslassungsleitung (22) und in den Formhohlraum der Ausbildungstaschen (10) sprüht.

9. Einrichtung (1), die zum Ausbilden geformter Ablagerungsstrukturen aus absorbierendem Material nach Anspruch 8 geeignet ist, wobei die Zufuhreinheit (4) zwei Auslassungsleitungen (22a,22b) umfasst, wobei jede Leitung (22a,22b) mit der Zerfaserungsstation (12) fluidisch verbunden ist und jede Leitung (22a,22b) umfassend ein Auslassrohr (32a,32b), das sich teilweise innerhalb seiner jeweiligen Auslassungsleitung (22a,22b) erstreckt, wobei die Ablaufdüse jeder Leitung (32a,32b) innerhalb ihrer jeweiligen Auslassungsleitung (22a,22b) eingerichtet ist.

**10.** Einrichtung (1), die zum Ausbilden geformter Ablagerungsstrukturen aus absorbierendem Material nach einem der Ansprüche 8 oder 9 geeignet ist, wobei sich die Einrichtung (1) in Längsrichtung, Querrichtung und Vertikalrichtung (L, **T,** V) erstreckt, wobei der Umlenkabschnitt (46) und die obere Wand des Gehäuses (26), die die Auslassungsleitung (22) definiert, sich in parallelen Ebenen erstreckt, wobei die Ebenen durch die Längs- und Querrichtung definiert sind.

**11.** Einrichtung (1), die zum Ausbilden geformter Ablagerungsstrukturen aus absorbierendem Material nach einem der Ansprüche 8 bis 10 geeignet ist, wobei das Auslassrohr (32) und die obere Wand des Gehäuses (26), die die Auslassungsleitung (22) definiert, einen zweiten Winkel ($\alpha$2) definieren, wobei der zweite Winkel ($\alpha$2) zwischen 0° und 90°, vorzugsweise zwischen 10° und 60° umfasst ist.

**12.** Einrichtung (1), die zum Ausbilden geformter Ablagerungsstrukturen aus absorbierendem Material nach einem der Ansprüche 8 bis 11 geeignet ist, wobei das Auslassrohr (32) innerhalb der Auslassungsleitung (22) auf eine Weise eingerichtet ist, dass der Abstand (D1) zwischen dem distalen Ende des Umlenkabschnitts (46) und der oberen Wand des Gehäuses (26) in Bezug auf die Vertikalrichtung (V) zwischen 20 und 80 mm umfasst ist und/oder der kürzeste Abstand (D2) zwischen dem distalen Ende des Umlenkabschnitts (46) und der Außenoberfläche der Ausbildungswalze (6), vorzugsweise der Außenoberfläche der Ausbildungstaschen (10), in Bezug auf die Längsrichtung (L) zwischen 100 und 1200 mm umfasst ist.

**13.** Einrichtung (1), die zum Ausbilden geformter Ablagerungsstrukturen aus absorbierendem Material nach einem der Ansprüche 8 bis 12 geeignet ist, wobei die Einrichtung (1) eine Ausrichtungsvorrichtung (52) für das Auslassrohr umfasst, die es ermöglicht, die Neigung des Auslassrohrs (32) zu ändern.

**14.** Verfahren zum Ausbilden geformter Ablagerungsstrukturen aus absorbierendem Material, die als absorbierende Kerne für absorbierende Artikel unter Verwendung einer Einrichtung nach einem der Ansprüche 8 bis 13 verwendet werden sollen, das Verfahren umfassend:

a) einen Zerfaserungsschritt, wobei eine kontinuierliche Lage aus absorbierendem Material (14) durch eine Zerfaserungsmaschine (16) an einer Zerfaserungsstation (12) zerfasert wird, wobei dadurch eine Vielzahl von absorbierenden Fasern (18) erhalten wird;
b) einen Transportschritt, wobei die Vielzahl der absorbierenden Fasern (18) durch eine zweite Luftströmung (24), die innerhalb der Auslassungsleitung (22) strömt, von der Zerfaserungsstation (12) zu der Ausbildungstasche (10) befördert wird;
c) einen Zufuhrschritt, wobei eine bestimmte Menge an superabsorbierenden Partikeln (34) zusammen mit der ersten Luftströmung (62) aus dem Auslassrohr (32) über die Ablaufdüse (40) in die Auslassungsleitung (22) gesprüht wird;
d) einen Ansammlungsschritt, wobei die absorbierenden Fasern (18) und die superabsorbierenden Partikel (34) sich innerhalb des Formhohlraums der Ausbildungstasche (10) ansammeln, wobei dadurch geformte Ablagerungsstrukturen aus absorbierendem Material ausgebildet werden, die als absorbierende Kerne für absorbierende Artikel verwendet werden sollen.

## Revendications

**1.** Appareil de distribution de particules superabsorbantes (34) sur un substrat, l'appareil comprenant un tuyau d'évacuation (32) pour acheminer un courant (35) comprenant des particules superabsorbantes (34) et un premier flux d'air (62), ledit tuyau d'évacuation (32) comprenant une section d'acheminement (38) et une buse de sortie (40), ladite buse de sortie (40) comprenant une partie droite (42) et une partie de déviation (46) **caractérisé en ce que** la partie de déviation (46) comprend des moyens de déviation (48) agencés de manière à ce que seule une partie (35a) du courant (35) de particules superabsorbantes (34) et du premier flux d'air (62) soit déviée et que l'autre partie (35b) dudit courant ne soit pas déviée, créant ainsi un flux croisé entre la partie dudit courant (35a) qui est déviée et la partie dudit courant (35b) qui n'est pas déviée.

**2.** Appareil de distribution de particules superabsorbantes (34) sur un substrat selon la revendication 1, dans lequel les moyens de déviation (48,48a,48b) définissent un premier angle ($\alpha$1) entre la section d'acheminement (38) et la partie de déviation (46), ledit premier angle ($\alpha$1) étant compris entre 91° et 179°, de préférence entre 120° et 175°.

**3.** Appareil de distribution de particules superabsorbantes (34) sur un substrat selon l'une quelconque des revendications précédentes, dans lequel le tuyau d'évacuation (32) s'étend longitudinalement, transversalement et verticalement (L, T, V), ladite buse de sortie (40) comprend une paroi supérieure (42), une paroi inférieure (46), une première et une seconde paroi latérale (45), chaque paroi comprenant une extrémité proximale reliée à la section d'acheminement (38) et une extrémité distale agencée au niveau de la sortie du tuyau d'évacuation (32), l'extrémité distale de la paroi supérieure (42) s'étendant longitudinalement au-delà de l'extrémité distale de la paroi inférieure (46) et/ou l'extrémité distale de la première paroi latérale (45) s'étendant longitudinalement au-delà de l'extrémité distale de la seconde paroi latérale (45).

4. Appareil de distribution de particules superabsorbantes sur un substrat selon l'une quelconque des revendications précédentes, dans lequel le tuyau d'évacuation (32) comprend en outre un premier tube d'alimentation (60) acheminant un premier flux d'air (62), un second tube d'alimentation (64) acheminant des particules superabsorbantes (34) et une section venturi (58) agencée au niveau de la jonction entre le premier et le second tube d'alimentation (60,64) et la section d'acheminement (38), le premier et le second tube d'alimentation (60,64) et la section d'acheminement (38) étant tous reliés fluidiquement, le premier et le second tube d'alimentation (60,64) étant agencés en amont de la section d'acheminement (38) par rapport à la direction du flux.

5. Appareil de distribution de particules superabsorbantes sur un substrat selon la revendication 4, dans lequel la section venturi (58) est formée par le second tube d'alimentation (64) s'étendant partiellement à l'intérieur du premier tube d'alimentation (60) et réduisant la surface de section transversale du tuyau d'évacuation (32) pour le premier flux d'air (62).

6. Appareil de distribution de particules superabsorbantes sur un substrat selon l'une quelconque des revendications 4 ou 5, dans lequel le premier tube d'alimentation (60) et le second tube d'alimentation (64) définissent un troisième angle (α3), ledit troisième angle (α3) étant compris entre 0° et 90°, de préférence entre 15° et 60°.

7. Appareil de distribution de particules superabsorbantes sur un substrat selon l'une quelconque des revendications précédentes, dans lequel la buse de sortie (40) comprend une section transversale rectangulaire et la surface de section transversale de la buse de sortie (40) est inférieure à la surface de section transversale de la section d'acheminement (38).

8. Appareil (1) approprié pour former des structures de dépôt de matériau absorbant moulé à utiliser comme noyaux absorbants pour des articles absorbants, l'appareil (1) comprenant :

   - une unité de formage (2) comprenant : un tambour de formage (6) comprenant une surface circonférentielle externe, une pluralité de poches de formage (10) agencées sur la surface circonférentielle externe dudit tambour de formage (6), chaque poche de formage (10) comportant une cavité de moule ; et
   - une unité d'alimentation (4) comprenant :

      ◦ une station de défibrage (12) conçue pour générer des fibres absorbantes (18) et un second flux d'air (24),
      ◦ un conduit d'évacuation (22) défini par un boîtier (26) reliant fluidiquement la station de défibrage (12) et l'unité de formage (2) à travers lequel s'écoulent la pluralité de fibres absorbantes (18) et un second flux d'air (24),
      ◦ un appareil de distribution de particules superabsorbantes (34) selon les revendications 1 à 7,
      dans lequel le tuyau d'évacuation (32) s'étend partiellement à l'intérieur du conduit d'évacuation (22), la buse de sortie (40) étant agencée à l'intérieur du conduit d'évacuation (22) de manière à ce que le tuyau d'évacuation (32) pulvérise des particules superabsorbantes (34) dans le conduit d'évacuation (22) et dans la cavité de moule des poches de formage (10).

9. Appareil (1) approprié pour former des structures de dépôt de matériau absorbant moulé selon la revendication 8, dans lequel l'unité d'alimentation (4) comprend deux conduits d'évacuation (22a,22b), chaque conduit (22a,22b) étant relié fluidiquement à la station de défibrage (12) et chaque conduit (22a,22b) comprenant un tuyau d'évacuation (32a,32b) s'étendant partiellement à l'intérieur de son conduit d'évacuation respectif (22a,22b), la buse de sortie de chaque tuyau (32a,32b) étant agencée à l'intérieur de son conduit d'évacuation respectif (22a,22b).

10. Appareil (1) approprié pour former des structures de dépôt de matériau absorbant moulé selon l'une quelconque des revendications 8 ou 9, dans lequel l'appareil (1) s'étend longitudinalement, transversalement et verticalement (L, **T**, V), la partie de déviation (46) et la paroi supérieure du boîtier (26) définissant le conduit d'évacuation (22) s'étendent dans les plans parallèles, lesdits plans étant définis par les directions longitudinale et transversale.

11. Appareil (1) approprié pour former des structures de dépôt de matériau absorbant moulé selon l'une quelconque des revendications 8 à 10, dans lequel le tuyau d'évacuation (32) et la paroi supérieure du boîtier (26) définissant le conduit d'évacuation (22) définissent un deuxième angle (α2), ledit deuxième angle (α2) étant compris entre 0° et 90°, de préférence entre 10° et 60°.

12. Appareil (1) approprié pour former des structures de dépôt de matériau absorbant moulé selon l'une quelconque des revendications 8 à 11, dans lequel le tuyau d'évacuation (32) est agencé à l'intérieur du conduit d'évacuation (22) de manière à ce que la distance (D1) entre l'extrémité distale de la partie de déviation (46) et la paroi supérieure du boîtier (26) par rapport à la direction verticale (V) soit comprise entre 20 et 80 mm et/ou la distance la plus courte (D2) entre l'extrémité distale de la partie de déviation (46) et la surface extérieure du tambour de formage (6), de préférence, la surface extérieure des poches de formage (10) par rapport à la direction longitudinale (L) soit comprise entre 100 et 1200 mm.

13. Appareil (1) approprié pour former des structures de dépôt de matériau absorbant moulé selon l'une quelconque des revendications 8 à 12, dans lequel l'appareil (1) comprend un dispositif d'orientation du tuyau d'évacuation (52) permettant de modifier l'inclinaison du tuyau d'évacuation (32).

14. Procédé permettant de former des structures de dépôt de matériau absorbant moulé à utiliser comme noyaux absorbants pour des articles absorbants à l'aide d'un appareil selon l'une quelconque des revendications 8 à 13, le procédé comprenant :

a) une étape de défibrage, dans lequel une feuille continue de matériau absorbant (14) est défibrée par une machine de défibrage (16) au niveau d'une station de défibrage (12), ce qui permet d'obtenir une pluralité de fibres absorbantes (18) ;
b) une étape d'acheminement, dans lequel ladite pluralité de fibres absorbantes (18) sont acheminées par un second flux d'air (24) s'écoulant à l'intérieur du conduit d'évacuation (22) de la station de défibrage (12) à la poche de formage (10) ;
c) une étape d'alimentation, dans lequel une quantité spécifique de particules superabsorbantes (34) en conjonction avec le premier flux d'air (62) est pulvérisée du tuyau d'évacuation (32) dans le conduit d'évacuation (22) par l'intermédiaire de la buse de sortie (40) ;
d) une étape d'accumulation, dans lequel les fibres absorbantes (18) et les particules superabsorbantes (34) s'accumulent au sein de la cavité de moule de la poche de formage (10), ce qui permet de former des structures de dépôt de matériau absorbant moulé à utiliser comme noyaux absorbants pour des articles absorbants.

**FIG. 1**

**FIG. 2**

FIG. 3

FIG. 4

**FIG. 5**

**FIG. 6**

FIG. 7

FIG. 8

FIG. 9

**EP 4 378 436 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050234412 A
- US 20110166541 A
- US 20140027943 A
- JP 2009112347 A